# EUROPEAN PATENT SPECIFICATION

(11) **EP 3 847 964 B1**
(45) Date of publication and mention of the grant of the patent: **27.09.2023**
(21) Application number: 19857057.4
(22) Date of filing: 23.08.2019
(51) Int. Cl.: A61B 5/145, A61B 5/00

(54) **BIOLOGICAL INFORMATION MEASUREMENT DEVICE**
BIOLOGISCHE INFORMATIONSMESSVORRICHTUNG
DISPOSITIF DE MESURE D'INFORMATIONS BIOLOGIQUES

(30) Priority: 05.09.2018 JP 2018166173; 05.09.2018 JP 2018166175
(43) Date of publication of application: 14.07.2021
(73) Proprietor: PHC Holdings Corporation, Tokyo 105-8433 (JP)
(72) Inventor: KOUGE, Masahiro, Ehime 791-0395 (JP); IKETANI, Seiichiro, Ehime 791-0395 (JP); NISHIO, Akira, Ehime 791-0395 (JP); OKAZAKI, Makoto, Ehime 791-0395 (JP); SHIMIZU, Ryuji, Ehime 791-0395 (JP)
(74) Representative: Eisenführ Speiser
(86) International application number: PCT/JP2019/033024
(87) International publication number: WO 2020/050060

(56) References cited:
- WO-A1-2010/109844
- JP-A- 2006 145 974
- JP-A- 2012 098 258
- JP-A- 2015 093 167
- JP-A- 2015 509 011
- JP-A- 2016 128 031
- JP-U- H0 638 906
- JP-U- S59 116 007
- US-A1- 2011 279 963
- US-A1- 2014 094 676
- US-A1- 2017 290 535

## Description

### TECHNICAL FIELD

The present invention relates to a biological information measurement device, a biological information measurement system used for continuous blood glucose measurement, for example, and an inserter.

### BACKGROUND ART

An electronic device for long-term adhesion to a mammal according to US 2011/0279963 A1 includes a housing with an electronic component. There is a first wing and a second wing, each being integrally formed with the housing. An electrode is positioned on a bottom surface of each of the wings, the electrodes electrically connected to the electronic component. An adhesive layer is provided for adhesion to a surface of the mammal. The adhesive layer is coated on a portion of the bottom surfaces of the wings. The adhesive layer is not coated on the electrode or on a bottom surface of the housing. A method of applying an electronic device to a mammal includes removing a first adhesive cover from a first wing of the electronic device to expose an electrode and an adhesive coated on a bottom surface of the first wing. There is a step of placing the exposed electrode into contact with the mammal by adhering the adhesive coated bottom of the first wing to the mammal. There is a step of removing a second adhesive cover from the second wing of the electronic device to expose an adhesive coated on a bottom surface of the second wing and another exposed electrode. There is a step of placing the another exposed electrode into contact with the mammal by adhering the adhesive coated bottom of the second wing to the mammal. After performing the removing and the placing steps, the housing is unattached to the mammal, but is held in position on the mammal using the adhesive coated bottoms of the first and the second wings.

Wearable (ambulatory) monitors according to US 2014/0094676 A1 have a segmented design, with at least two and preferably three (or more) mechanically independent, spaced apart sensors (e.g., electrodes or other types of physiological sensors) located in discrete islands, or housing structures that are flexibly connected to one another. Each of the sensors is in operable communication with one or more electronics module(s), also located in one or more of the islands. In some embodiments, an electronics module may be centrally located with respect to two or more peripherally located sensors.

A sensing device according to US 2017/0290535 A1 includes a case with one or more indicators to indicate levels of an analyte such as glucose. The sensing device for self-monitoring an analyte includes a single, flexible case adapted to adhere to a skin of a patient. It also includes a printed circuit board assembly inside the case. It further includes a first sensor extending from the flexible case and electrically coupled to the printed circuit board, and one or more indicators in the flexible case, where the indicator(s) are adapted to indicate whether a level of an analyte is within a normal range.

A conventional biological information measurement device comprised an adhesive tape having on its bottom side an adhesive surface that was affixed to the skin of user, a mounting substrate that was on the top side of the adhesive tape, and a sensor that was electrically connected to the mounting substrate and a part of which was transferred to a side of the skin of user (see, for example, Patent Literature 1 below).

Also, a conventional biological information measurement system comprised an adhesive tape, a mounting substrate disposed on the top side of the adhesive tape, a sensor electrically connected to the mounting substrate, and a cover that covered the top side of the mounting substrate. The configuration was such that the biological information measurement device was housed within an inserter, and the biological information measurement device was mounted on the human body by operating this inserter (see, for example, Patent Literature 1 below).

### CITATION LIST

### PATENT LITERATURE

Patent Literature 1: US Patent Application Publication No. 2017/0027514
Patent Literature 2: Japanese Unexamined Patent Application Publication No. 2015-509011

### SUMMARY

### TECHNICAL PROBLEM

In Patent Literature 1, a flexible substrate is used as a mounting substrate. Therefore, the flexible substrate bends to conform to the shape of the body part to which the biological information measurement device is attached, and this is anticipated to make the attachment to the skin more comfortable. After this, the flexible substrate and adhesive tape are covered with the cover.

However, with the above-mentioned conventional configuration, improved comfort cannot be expected during use beyond the initial attachment. The reason for this is that when the mounting substrate is a flexible substrate, the flexible substrate bends to conform to the shape of the attachment portion when attached to the user's body. Accordingly, at the time of attachment, the biological information measurement device is worn in a state of being fitted to the body, but the skin, bones, and muscles of the body move around considerably in the course of daily activities, so the flexible substrate was bent into one shape at the time of attachment may inhibit the displacement of the underlying skin. For instance, the flexible substrate inhibit displacement of the skin when the skin tries to stretch, and this can end up causing discomfort to the user, so improved comfort is desirable.

### SOLUTION TO PROBLEM

In view of this, a first object of the first invention is to improve the comfort of wearing a biological information measurement device.

In order to achieve the first object, a biological information measurement is provided as defined in claim 1.

With this configuration, the first object is achieved.

The disclosure also generally relates to a biological information measurement system comprising a biological information measurement device that measures biological information about a user, and an inserter that is placed

### ADVANTAGEOUS EFFECTS

As described above, in the invention, the adhesive tape affixed to the skin of user and the cover on the top side are stretchable, and the mounting substrate located between the adhesive tape and the cover is held by the substrate holding portion provided on the top side of the adhesive tape. Furthermore, since the bottom side of the mounting substrate is not bonded to the top side of the adhesive tape, the biological information measurement device is more comfortable when worn on the skin.

That is, since a very stretchable adhesive tape and cover are used, when the biological information measurement device is attached to the skin, it can be attached in a state of conforming to the skin. Also, if the body should be displaced (move) after attachment, stretching of the adhesive tape and the cover will minimize discomfort to the wearer, so the biological information measurement device can be worn more comfortably than a conventional device.

### BRIEF DESCRIPTION OF DRAWINGS

FIG. 1 is an oblique view of the packaged state of the biological information measurement device according to Embodiment 1 of the present invention;
FIG. 2 is an oblique view of the biological information measurement device in FIG. 1;
FIG. 3 is a cross section of the biological information measurement device in FIG. 1;
FIG. 4 is a cross section of the biological information measurement device in FIG. 1;
FIG. 5 is a cross section of the biological information measurement device in FIG. 1;
FIG. 6 is a cross section of the biological information measurement device in FIG. 1;
FIG. 7 is an exploded oblique view of the biological information measurement device in FIG. 1;
FIG. 8 is an exploded oblique view of the biological information measurement device in FIG. 1;
FIG. 9 is an oblique view of the usage state of the biological information measurement device in FIG. 1;
FIG. 10 is an oblique view of the usage state of the biological information measurement device in FIG. 1;

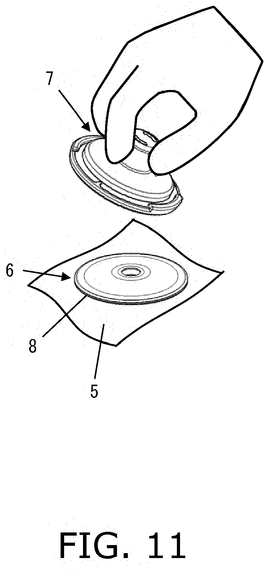

FIG. 11 is an oblique view of the usage state of the biological information measurement device in FIG. 1;
FIG. 12 is an oblique view of the usage state of the biological information measurement device in FIG. 1;
FIG. 13 is a cross section of the usage state of the biological information measurement device in FIG. 1;
FIG. 14 is an exploded oblique view of the biological information measurement device in FIG. 1;
FIG. 15 is a detail cross section of the biological information measurement device in FIG. 1;
FIG. 16 is a cross section of the usage state of the biological information measurement device in FIG. 1;
FIG. 17 is a cross section of the usage state of the biological information measurement device in FIG. 1;
FIG. 18 is an oblique view of the usage state of the biological information measurement device in FIG. 1;
FIG. 19 is an oblique view of the usage state of the biological information measurement device in FIG. 1;
FIG. 20 is a cross section of the usage state of the biological information measurement device in FIG. 1;
FIG. 21 is an oblique view of the usage state of the biological information measurement device in FIG. 1;
FIG. 22 is a cross section of the biological information measurement device in FIG. 1;
FIG. 23 is an oblique view of an overtape of the biological information measurement device in FIG. 1;
FIG. 24 is a cross section of the biological information measurement device in FIG. 1 in a state where an overtape has been attached;
FIG. 25 is an oblique view of the state when the biological information measurement device in FIG. 1 is attached; and
FIG. 26 is an electrical circuit diagram of the biological information measurement device in FIG. 1.

### DESCRIPTION OF EMBODIMENTS

Embodiments of the present invention will now be described in detail with reference to the drawings.

### Embodiment 1

A biological information measurement system 1A including a biological information measurement device 6 and an inserter 7 of this embodiment will now be described with reference to the drawings.

In the following description, terms indicating a specific direction or position are used (such as "top," "bottom," "right," "left," as well as other terms including those terms), but the use of these terms is intended to facilitate an understanding of the disclosure with reference to the drawings, and the meaning of these terms is not intended to limit the technical scope of the invention.

### (1) Configuration of Biological Information Measurement System 1A

The biological information measurement system 1A of this embodiment is used, for example, to attach the biological information measurement device 6 that measures blood glucose level and other such biological information to a user's upper arm or the like by using the inserter 7.

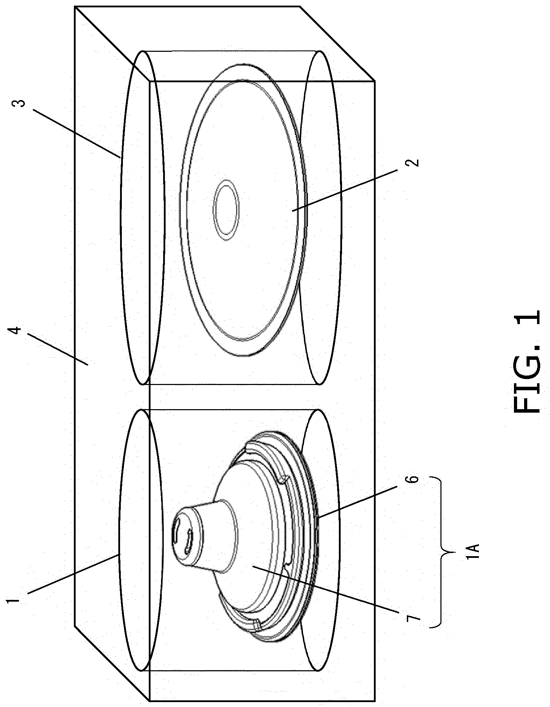

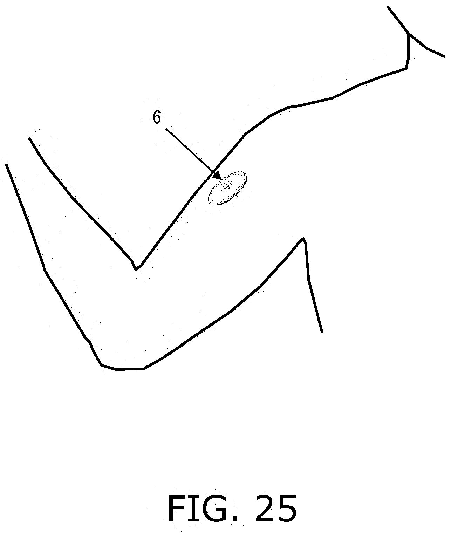

FIG. 1 shows a state in which a sterilized packaging 1 containing the biological information measurement device 6 and the inserter 7, and a sterilized packaging 3 containing an overtape 2 are housed in a container 4. That is, the biological information measurement device is transported while kept in a sterile state in the container 4 as shown in FIG. 1 before being attached to the skin 5 of the upper arm of the user whose biological information is to be measured, as shown in FIG. 25. In this embodiment, the biological information measurement system 1A is constituted by the biological information measurement device 6 and the inserter 7.

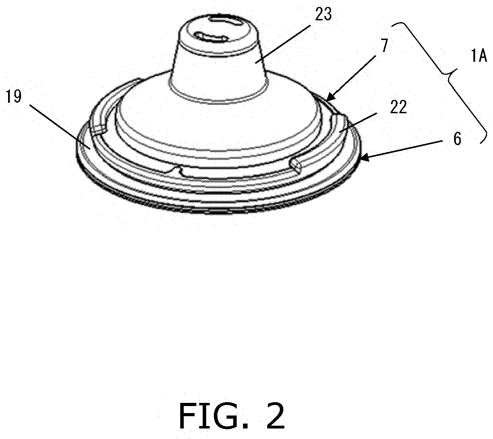

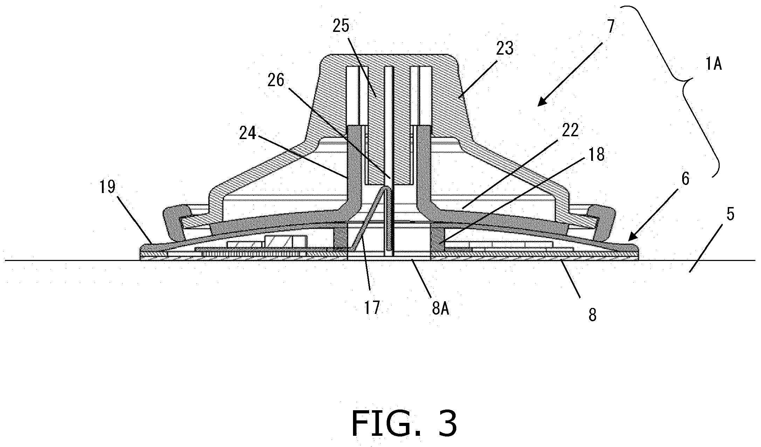

FIGS. 2 and 3 show the biological information measurement device 6 and the inserter 7 constituting the biological information measurement system 1A.

As shown in FIGS. 2 and 3, the inserter 7 used for attaching the biological information measurement device 6 to the upper arm, etc., of the user is detachably mounted on the upper surface of the biological information measurement device 6. Then, as mentioned above, the biological information measurement device 6 and the inserter 7 are held in the sterilized packaging 1 before use, as shown in FIG. 1.

### (2-1) Configuration of Biological Information Measurement Device 6

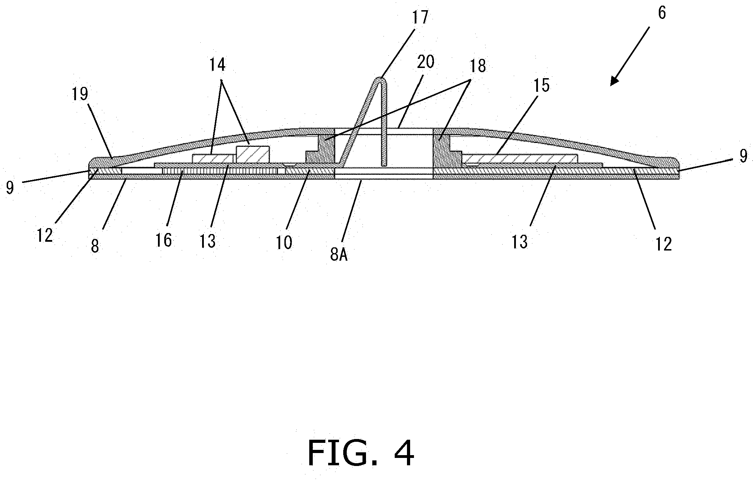

The biological information measurement device 6 has the configuration shown in FIGS. 4 to 8.

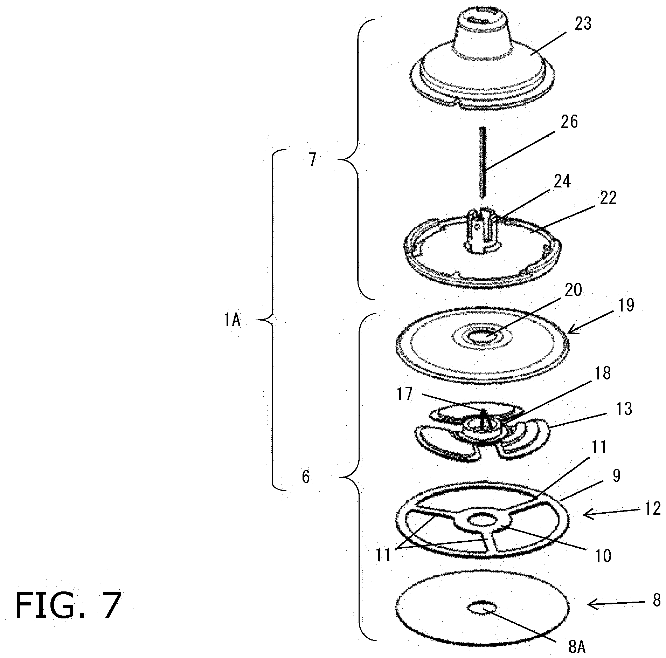

That is, as shown in FIGS. 4 and 7, the biological information measurement device 6 comprises an adhesive tape 8, a base 12, a mounting substrate 13, a sensor 17, a guide needle insertion cylinder 18 (an example of a substrate holding portion), and a cover 19.

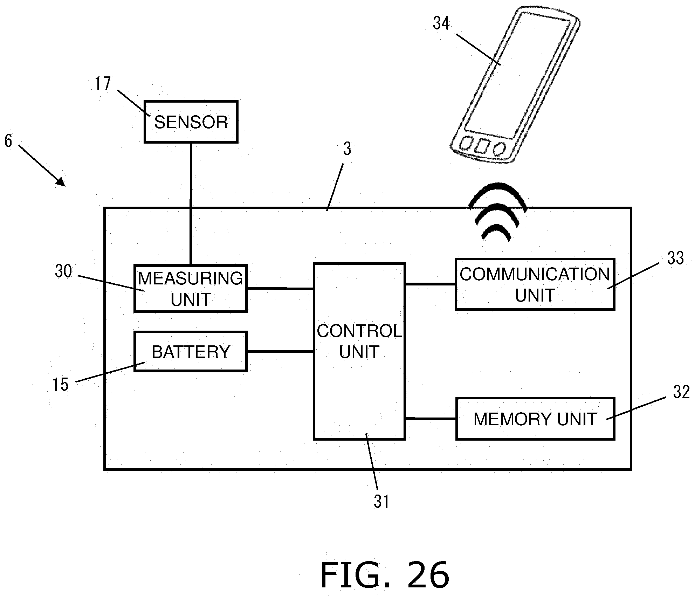

FIG. 26 is an electrical block diagram of the biological information measurement device 6.

The measured value obtained by a measuring unit 30 using the sensor 17 is stored in a memory unit 32 via a control unit 31. Also, the measured value stored in the memory unit 32 is transmitted through a communication unit 33 to a mobile device 34.

As shown in FIG. 7, the substantially circular adhesive tape 8 is provided on the skin 5 side of the user's body in the biological information measurement device 6. The adhesive tape 8 is formed from a stretchable material such as rubber, an elastomer, a non-woven fabric, or a pressure-sensitive adhesive material. A through-hole 8A is provided in the center of the adhesive tape 8.

A base 12 in which the outer peripheral circle portion 9 and the inner peripheral circle portion 10 are connected by bridge portions 11 at intervals of 120 degrees as shown in FIG. 7 is provided on the adhesive tape 8.

The outer peripheral circle portion 9 forms the outermost peripheral portion of the substantially annular base 12, and is disposed concentrically with the inner peripheral circle portion 10. Also, the outer peripheral circle portion 9 and the inner peripheral circle portion 10 are connected to each other by three bridge portions 11 disposed in the radial direction.

The lower surface of the adhesive tape 8 is an entirely adhesive surface. On the other hand, the upper surface of the adhesive tape 8 is provided with an adhesive surface only on the outer peripheral circle portion 9, the inner peripheral circle portion 10, and the bridge portions 11. Consequently, the base 12 is held in a state of being bonded to the adhesive tape 8.

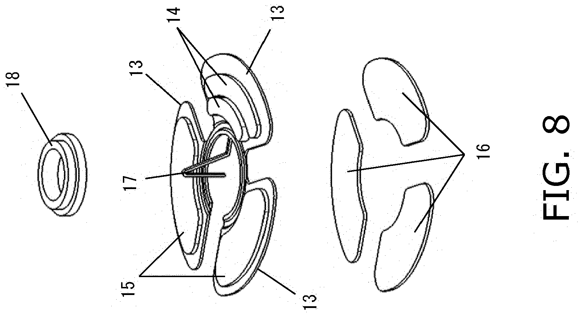

Next, as shown in FIGS. 4 and 7, the mounting substrate 13 is bonded onto the inner peripheral circle portion 10 of the base 12. As shown in FIG. 8, the mounting substrate 13 is divided into three parts at intervals of substantially equal angles (120 degrees). Electronic components 14 and a battery 15 are disposed on the upper surfaces of the mounting substrate 13 divided into three parts. As shown in FIG. 7, the mounting substrate 13 divided into three parts is disposed between the bridge portions 11 disposed at intervals of 120 degrees on the base 12.

More precisely, the three parts of the mounting substrate 13 are substantially fan shaped, each extending over an angular range of slightly less than 120 degrees. Therefore, between the bridge portions 11, the three parts of the mounting substrate 13 are disposed in a state of non-contact with the bridge portions 11, so as not to overlap the bridge portions 11 in top view.

Consequently, the spacer 16 disposed on the back side of the mounting substrate 13 can be disposed so as not to interfere with and ride up onto the bridge portions 11.

The mounting substrate 13 is formed from a resin such as polyimide or polyester used for flexible substrates. These materials are flexible in the bending direction, but they are not stretchable in a direction substantially parallel to the substrate surface after being attached to the skin 5.

Also, as shown in FIG. 8, fan-shaped spacers 16 are bonded to the bottom side of the three parts of the mounting substrate 13, that is, to the surface on the adhesive tape 8 side. Specifically, the mounting substrate 13 is in a state of being disposed on the adhesive tape 8 in an unbonded state via the spacers 16. That is, when the mounting substrate 13 is bonded, the adhesive tape 8 is less likely to be affected by the mounting substrate 13, which has no stretchability in the direction along the surface of the skin 5, which would hinder the stretching of the adhesive tape 8.

Also, the base 12 is formed from a stretchable material such as rubber or an elastomer. The base 12 is formed by connecting the outer peripheral circle portion 9 and the inner peripheral circle portion 10 to each other via the bridge portions 11 at intervals of 120 degrees as shown in FIG. 7. Accordingly, even when the base 12 is bonded to the upper surface of the adhesive tape 8, the stretching of the adhesive tape 8 will not be hindered by the stretchable base 12.

As shown in FIG. 4, the sensor 17 is electrically connected to the mounting substrate 13. The central portion of the sensor 17 is bent upward, and the distal end of the leg portion of the sensor 17 is held in a state of being inserted into the through-hole 8A in the adhesive tape 8.

The guide needle insertion cylinder 18 (an example of a substrate holding portion) is adhesively fixed to the top side of the mounting substrate 13 corresponding to the inner peripheral circle portion 10 of the base 12. That is, the mounting substrate 13 is held on the outer periphery of the guide needle insertion cylinder 18. The guide needle insertion cylinder 18 has a cylindrical shape. The central axis of the guide needle insertion cylinder 18 is disposed in a state of coinciding with the central axis of the through-hole 8A in the adhesive tape 8. As shown in FIG. 4, the central portion of the sensor 17 is disposed inside the guide needle insertion cylinder 18.

The guide needle insertion cylinder 18 is from a resin or the like, for example. That is, the guide needle insertion cylinder 18 is preferably formed from a material that is harder than the adhesive tape 8, the base 12, the mounting substrate 13, and the cover 19.

Consequently, since the guide needle holding portion 25, which holds a guide needle 26, is formed from a hard material that is not readily deformed, the guide needle 26 will not move around when inserted into the skin, and this allows the guide needle 26 to be inserted into the skin 5 (discussed below) stably and accurately.

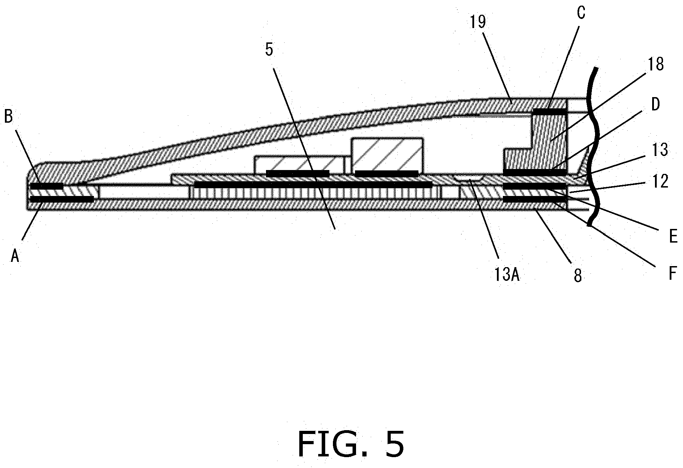

As shown in FIG. 5, the upper surface of the adhesive tape 8 and the lower surface of the base 12 are bonded together at a joint A of the outer peripheral edge portion. As shown in FIG. 5, the upper surface of the base 12 and the lower surface of the cover 19 are bonded together at a joint B of the outer peripheral edge portion. That is, the adhesive tape 8 and the cover 19 are bonded together via the base 12 at the outer peripheral edge portion.

As shown in FIG. 5, the lower surface of the cover 19 and the substantially cylindrical upper surface of the guide needle insertion cylinder 18 provided near the center of the substantially disk-shaped biological information measurement device 6 are bonded together at a position on the inner peripheral side (joint C).

The lower surface of the guide needle insertion cylinder 18 and the upper surface of the mounting substrate 13 are bonded together at a position on the inner peripheral side (joint D), similar to the joint C.

The lower surface of the mounting substrate 13 and the upper surface of the base 12 are bonded together at a position below the joint D of the guide needle insertion cylinder 18 (joint E).

The upper surface of the adhesive tape 8 and the lower surface of the base 12 are bonded together at a position below the joint D of the guide needle insertion cylinder 18 (joint F).

That is, the adhesive tape 8, the cover 19, and the base 12 are bonded together at the outer peripheral edge portion and near the center position of the substantially disk-shaped biological information measurement device 6.

The mounting substrate 13 is bonded to the adhesive tape 8, the cover 19, and the base 12 via the guide needle insertion cylinder 18 in the vicinity of the central portion of the substantially disk-shaped biological information measurement device 6.

Furthermore, the cover 19 is disposed on the mounting substrate 13 as shown in FIG. 7. The cover 19 is formed from a stretchable material such as rubber or an elastomer. As shown in FIG. 7, a through-hole 20 having substantially the same inside diameter as the through-hole 8A is provided in the central portion of the cover 19.

The outer peripheral edge of the through-hole 20 on the bottom side is adhesively fixed to the upper end surface of the substantially cylindrical guide needle insertion cylinder 18. Also, the lower surface of the cover 19 on the outer peripheral side is adhesively fixed to the upper surface of the outer peripheral circle portion 9 of the base 12. The cover 19 is disposed in state of not being bonded to the mounting substrate 13. That is, the cover 19 is disposed in a state of being free to stretch, without being affected by the mounting substrate 13.

In this embodiment, the mounting substrate 13 is disposed on the adhesive tape 8 via the spacers 16 bonded to the bottom side. Therefore, the mounting substrate 13 is put in place without being bonded to the adhesive tape 8. Also, on the upper surface of the adhesive tape 8, an adhesive surface is provided only on the outer peripheral circle portion 9, the inner peripheral circle portion 10, and the bridge portions 11. Accordingly, the mounting substrate 13 and the spacers 16 are not bonded to the adhesive tape 8. Also, the mounting substrate 13 is put in place in a state of not being bonded to the lower surface of the cover 19.

Consequently, the adhesive tape 8 and the cover 19 can smoothly expand and contract without being affected by the mounting substrate 13. Also, when the adhesive tape 8 and the cover 19 are expanded and contracted, the mounting substrate 13 is not affected by the displacement of the adhesive tape 8 and the cover 19. This ensures good reliability of the mounting substrate 13.

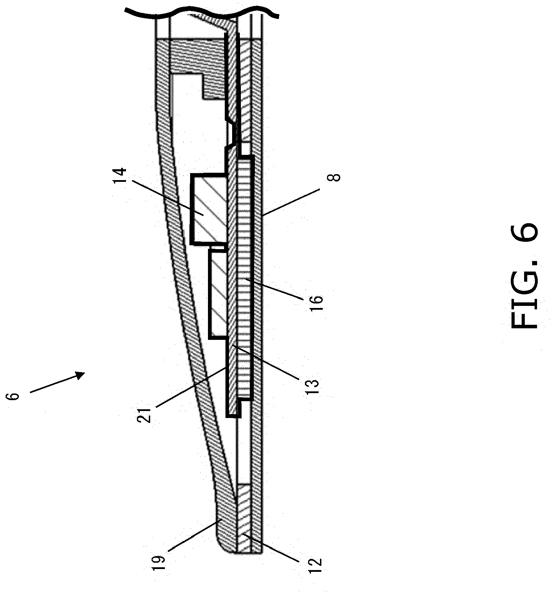

Furthermore, in this embodiment, as shown in FIG. 6, the mounting substrate 13 and the electronic components 14, the battery 15, and the spacers 16 mounted on the mounting substrate 13 are covered with a waterproof film 21. This minimizes degradation of the mounting substrate 13 due to moisture.

There is a risk that the waterproof films 21 covering the bottom side of the spacers 16 will be peeled off by friction when the adhesive tape 8 expands and contracts. However, the portion peeled off by friction will be on only the bottom side of the spacer 16, and no peeling will occur in the thickness direction or in the upper surface portion. This means that it is possible to prevent the function of the waterproof film 21 from being diminished. The spacers 16 are formed by using a flexible material such as an elastomer, rubber, or silicone.

### (2-2) Configuration of Inserter 7

The inserter 7 included in the biological information measurement system 1A in this embodiment is a device used for attaching the biological information measurement device 6 to the upper arm of the user, and as shown in FIGS. 2, 3, and 7, the inserter 7 is mounted on the upper surface of the biological information measurement device 6.

The inserter 7 comprises a substantially circular base body 22 and a circular container-shaped top body 23, in a state of being attached to the biological information measurement device 6.

The base body 22 covers the top side of the cover 19 of the biological information measurement device 6 and is supported by the upper end portion of the guide needle insertion cylinder 18 via the cover 19. The base body 22 has a guide cylinder 24 protruding toward the top body 23 from near the approximate center of the substantially circular upper surface.

The top body 23 is mounted on the base body 22 and is flexible. As shown in FIG. 3, the top body 23 has a substantially cylindrical guide needle holding portion 25 that can move up and down on the inner peripheral surface side of the guide cylinder 24.

The substantially cylindrical guide needle holding portion 25 holds the guide needle 26 along the center axis of the substantially cylindrical shape. When the central portion of the upper surface of the top body 23 is pressed toward the base body 22, the guide needle 26 transfers the sensor 17 to a side of the skin 5 of user. That is, the guide cylinder 24 provided to the base body 22 is provided in order to prevent the misalignment of the sensor 17 during transfer, and to perform positioning in the transfer of the guide needle holding portion 25.

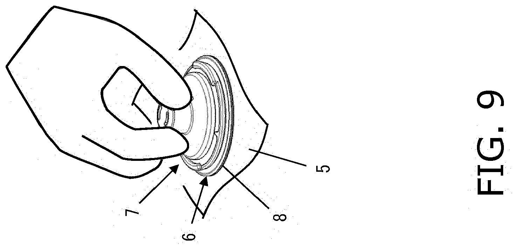

In the above configuration, the user first takes the biological information measurement device 6 to which the inserter 7 is mounted out of the container 4 shown in FIG. 1, and then makes use of the stickiness of the adhesive tape 8 to affix the biological information measurement device 6 to the skin 5 of the upper arm while the inserter 7 is still attached, as shown in FIG. 9.

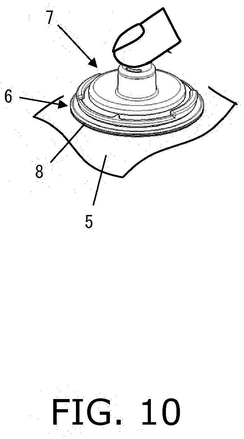

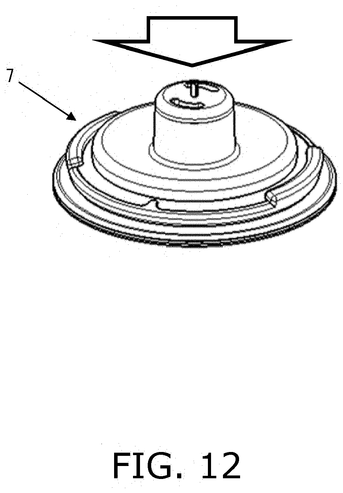

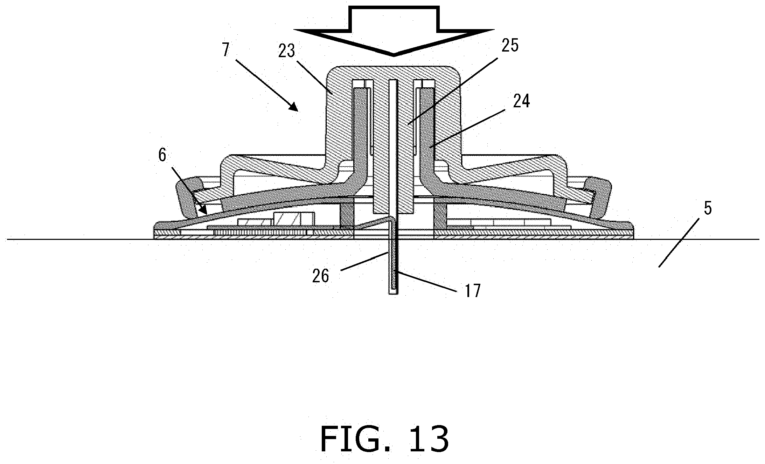

Next, as shown in FIGS. 10, 12, and 13, when the user pushes down the upper end surface of the inserter 7, the guide needle holding portion 25 of the inserter 7 slides through the guide cylinder 24 as shown in FIG. 13 and pushed down, that is, toward the skin 5. Consequently, the guide needle 26 pushes the sensor 17 toward the skin 5, which allows the distal end part of the sensor 17 to be transferred into the skin 5.

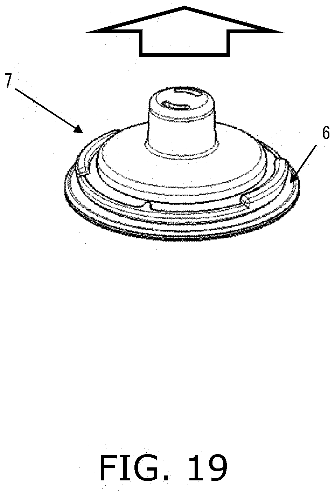

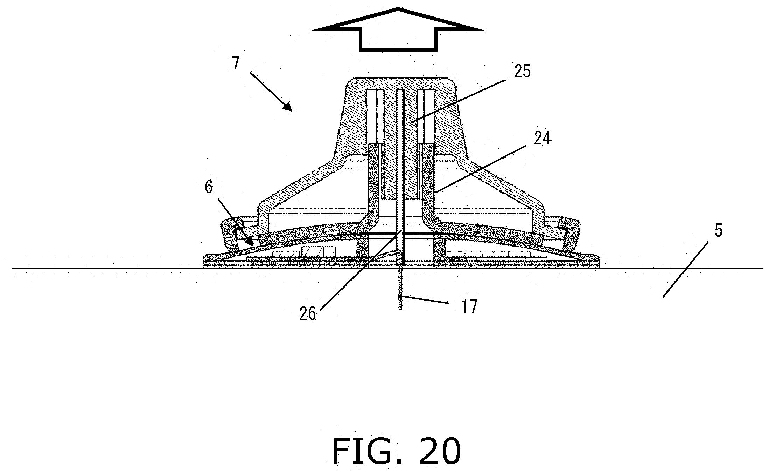

After this, the guide needle holding portion 25 of the inserter 7 automatically returns to its upper steady-state position under the elastic force (restoring force) of the inserter 7, as shown in FIGS. 19 and 20. This causes the distal end of the guide needle 26 that has been inserted into the skin 5 to be withdrawn from the skin 5.

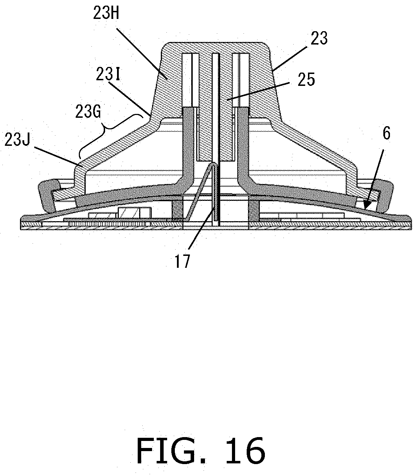

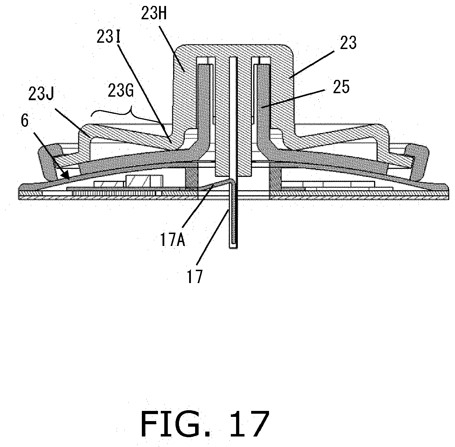

Also, as shown in FIGS. 16 and 17, in the top body 23, the wall thickness of a hinge portion 23G is less than the wall thickness of a pressing portion 23H, and the wall thickness of the hinge portion 23G is greater than the wall thickness of a bent portion 23I and a bent portion 23J.

Consequently, when the upper end surface of the top body 23 is pushed down, the thin hinge portion 23G is elastically deformed and pushed down, while the portion of the pressing portion 23H disposed on the outer peripheral side of the guide needle holding portion 25 hardly deforms at all due to its greater thickness.

Similarly, in the elastic deformation of the thin hinge portion 23G, the bent portions 23I and 23J, which are thicker than the hinge portion 23G, undergo almost no elastic deformation, and the pressing portion 23H is pushed down, with the bent portions 231 and 23J serving as fulcrums.

As a result, a stronger elastic force can be obtained for returning the top body 23 to its steady state, and the guide needle holding portion 25 can be operated more accurately and stably in the up and down direction.

Also, when the sensor 17 is inserted into the wound formed by puncturing the skin 5 with the guide needle 26 and the guide needle 26 is withdrawn from the skin 5 in the above operation, the sensor 17 is left behind under the skin 5.

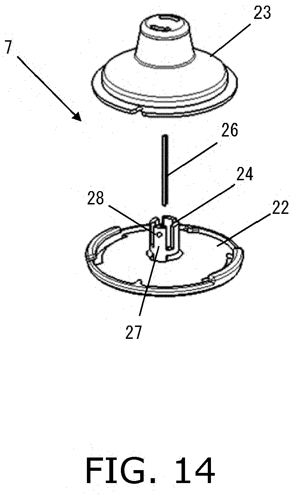

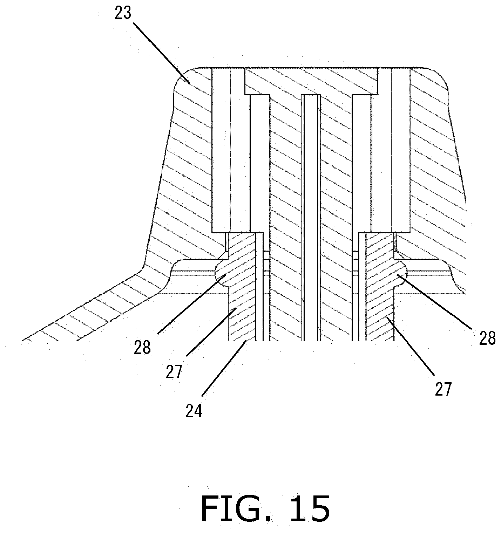

The inserter 7 in this embodiment has the configuration shown in FIGS. 14 and 15 in order for the guide needle holding portion 25 holding the guide needle 26 at the distal end to be pushed stably toward the skin 5.

Specifically, the guide cylinder 24 of the base body 22 has, on the upper end side thereof, a split piece 27 that is divided into four pieces in the circumferential direction. The split piece 27 has protrusions 28 that protrude in the outer peripheral direction (outward in the radial direction).

Therefore, when the top body 23 is pushed down, the protrusions 28 of the split piece 27 are pushed inward in the radial direction. Once the pressing force of this push operation overcomes the protrusions 28, it is quickly transmitted to the guide needle holding portion 25, so a constant pressing load can be ensured. Therefore, the guide needle 26 is reliably pushed in toward the skin 5.

In this embodiment, the configuration as shown in FIGS. 16 to 18 is employed so that when the sensor 17 is transferred under the skin 5 by pushing down the guide needle 26 in this way, the sensor 17 will not be broken by a sudden push on the guide needle 26.

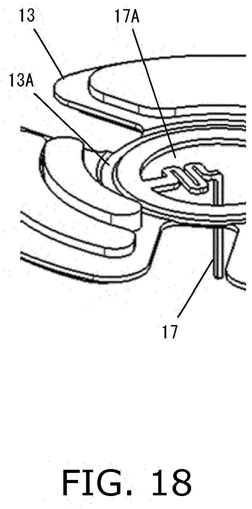

That is, as shown in FIG. 18, a meandering elastic portion 17A is provided near the portion of the sensor 17 that comes into contact with the distal end of the guide needle holding portion 25. Consequently, even if the distal end of the sensor 17 is rapidly pushed into the skin 5, it is possible to prevent a part of the sensor 17 from being damaged and the wire disconnected.

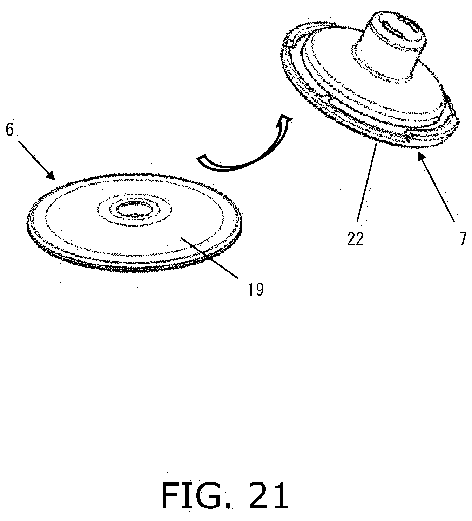

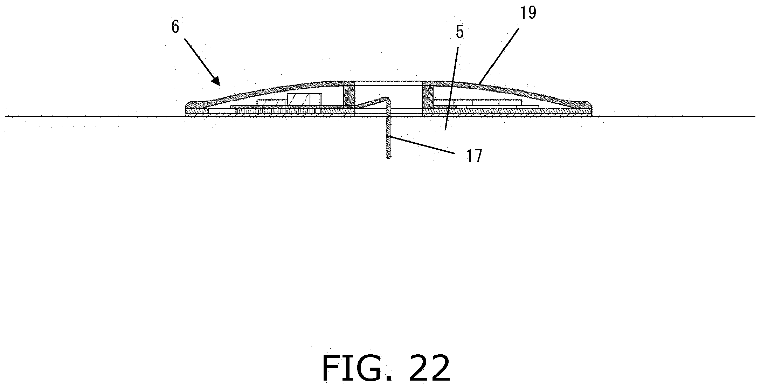

After this, as shown in FIG. 20, the distal end of the sensor 17 is transferred under the skin 5 and the top body 23 returns to its steady-state position under elastic force, after which the inserter 7 is removed from the upper surface (cover 19) of the biological information measurement device 6 as shown in FIGS. 21 and 22. That is, part of the base body 22 of the inserter 7 is bonded to the cover 19 in a state of point contact. This allows the inserter 7 to be easily peeled off from the biological information measurement device 6, as shown in FIG. 21.

At this point, the adhesive strength of the point contact portion between the cover 19 and the base body 22 of the inserter 7 is sufficiently less than the adhesive strength between the cover 19 and the guide needle insertion cylinder 18, the adhesive strength between the guide needle insertion cylinder 18 and the mounting substrate 13, the adhesive strength between the mounting substrate 13 and the base 12, the adhesive strength between the base 12 and the adhesive tape 8, and the adhesive strength between the adhesive tape 8 and the skin 5 shown in FIG. 5.

Consequently, as shown in FIG. 21, the inserter 7 can be easily peeled off from the biological information measurement device 6.

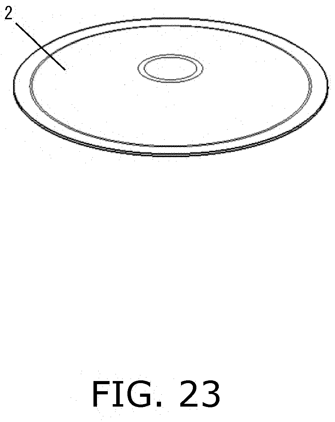

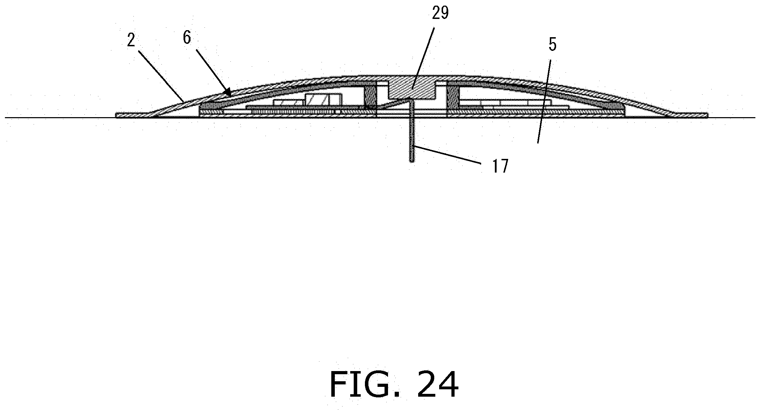

When the inserter 7 is removed from the biological information measurement device 6, the overtape 2 is attached to the top side of the cover 19 as shown in FIGS. 23 and 24.

The overtape 2 is formed from a stretchable material such as rubber or an elastomer. As shown in FIG. 24, a protrusion 29 that is inserted into the guide needle insertion cylinder 18 is provided to the portion of the overtape 2 corresponding to the guide needle insertion cylinder 18.

That is, the protrusion 29 comes into contact from above with the intermediate portion of the sensor 17 that the distal end of the guide needle holding portion 25 was in contact with when the biological information measurement device 6 was attached to the skin 5. This prevents the sensor 17 from inadvertently coming out upward.

The result of the above operation is that the biological information measurement device 6 is attached to the skin 5 of the upper arm of the user as shown in FIG. 25.

With the biological information measurement system 1A in this embodiment, the biological information measurement device 6 can be easily attached by hand to the skin 5 of the upper arm of the user by making use of the stickiness of the adhesive tape 8.

Also, in a state in which the biological information measurement device 6 to which the inserter 7 is attached has been affixed to the skin 5, the top body 23 is pushed in toward the skin 5 with the same hand used to affix the inserter 7, thereby allowing the sensor 17 to be easily transferred under the skin 5 by the guide needle 26.

Furthermore, after this, the inserter 7 bonded to the cover 19 of the biological information measurement device 6 by point contact can be easily removed by hand, and as shown in FIG. 24, the attachment of the biological information measurement device 6 to the skin 5 can be completed by affixing the overtape 2 on the upper surface of the biological information measurement device 6 by hand.

As shown in FIG. 24, after being attached, the biological information measurement device 6 has a shape that is thin and gradually thickens toward the central portion of the approximate disk shape. As a result, it is possible to avoid a shape in which the attached biological information measurement device 6 protrudes from the surface of the skin 5, so that the device is not unattractive when worn.

### Main Features

The adhesive tape 8, the cover 19, and the base 12 of the biological information measurement device 6 in this embodiment are formed from members that are stretchable in the direction running along the surface of the skin 5 and in the direction intersecting the surface of the skin 5 when the device is being worn, and are joined around the outer peripheral part. The mounting substrate 13, which does not stretch in the direction running along the surface of the skin 5, is fixed to the central portion of the adhesive tape 8, the cover 19, and the base 12 via the guide needle insertion cylinder 18.

Specifically, when the biological information measurement device 6 is attached to the skin 5, the adhesive tape 8, the cover 19, and the overtape 2 of the biological information measurement device 6 are pliant and stretchable.

Consequently, the biological information measurement device 6 can be attached in a state of conforming to the skin 5 to which the device is attached, which affords a biological information measurement device 6 that is more comfortable to wear.

Also, since the outer peripheral edge portions can be stretched out and attached to the skin 5 with respect to the central portion of the adhesive tape 8, the cover 19, and the overtape 2, there is stretchability in the direction running along the surface of the skin 5 and flexibility in the direction intersecting the surface of the skin 5, so the fit feels better when the device is attached.

Furthermore, in this embodiment, since the adhesive tape 8, the cover 19, and the overtape 2 can expand and contract according to the movements of the wearer's body during actual use, the user is less likely to feel any discomfort during body movements, and this further enhances wearing comfort.

In order to obtain this effect, as shown in FIG. 4, it is preferable for the taller of the battery 15 and the electronic components 14 to be mounted on the mounting substrate 13 at a position closer to the guide needle insertion cylinder 18.

As described above, the adhesive tape 8, the cover 19, and the base 12 in this embodiment are formed from a material, such as an elastomer, that is stretchable in the direction running along the surface of the skin 5, and in the direction intersecting the skin 5.

On the other hand, as described above, the mounting substrate 13 is formed from a polyimide or polyester resin used for a flexible substrate. These materials are flexible in the bending direction, but are not stretchable in a direction substantially parallel to the substrate surface.

The adhesive tape 8, the cover 19, the base 12, and the mounting substrate 13 are flexible in the bending direction. The mounting substrate 13 is placed on the adhesive tape 8 in an unbonded state via the spacers 16.

Consequently, the device can be affixed so that it fits the curves of the skin 5 where the device is attached, without being affected by the mounting substrate 13.

In this embodiment, as shown in FIG. 5, the mounting substrate 13 has a thin portion 13A provided around the periphery of the guide needle insertion cylinder 18, and near the guide needle insertion cylinder 18 (see FIG. 18). This allows the mounting substrate 13 to be bent more flexibly in the bending direction intersecting the surface of the substrate.

Also, the adhesive tape 8, the cover 19, and the base 12 are stretchable in a direction parallel to the surface of the skin 5 when the device is attached. This allows them to be affixed to the bonding site while being stretched out to both sides like a bandage tape.

At this point, the mounting substrate 13, which is not stretchable in the direction parallel to the surface of the skin 5 in the attached state, is fixed only in the central parts of the adhesive tape 8, the cover 19 and the base 12, which are stretchable in the direction parallel to the surface of the skin 5 in the attached state.

Consequently, the mounting substrate 13 will not be stretched out in the horizontal direction even when it is stretched out to both sides and attached to the skin 5.

Also, bending in the up and down direction is possible, matching the curved shape of the surface of the skin 5 to which the device is attached, the biological information measurement device 6 can be attached in a state of being fitted to the surface of the skin 5, similar to how a bandage tape is attached.

### INDUSTRIAL APPLICABILITY

The present invention is expected to find use as a biological information measurement device for continuous blood glucose measurement, for example.

### REFERENCE SIGNS LIST

- 1A: biological information measurement system
- 1: sterilized packaging
- 2: overtape
- 3: sterilized packaging
- 4: container
- 5: skin
- 6: biological information measurement device
- 7: inserter
- 8: adhesive tape
- 8a: through-hole
- 9: outer peripheral circle portion
- 10: inner peripheral circle portion
- 11: bridge portions
- 12: base
- 13: mounting substrate
- 13A: thin portion
- 14: electronic component
- 15: battery
- 16: spacer
- 17: sensor
- 17A: elastic portion
- 18: guide needle insertion cylinder (example of substrate holding portion)
- 19: cover
- 20: through-hole (through-hole)
- 21: waterproof film
- 22: base body
- 23: top body
- 23G: hinge portion
- 23H: pressing portion
- 23I: bent portion
- 23J: bent portion
- 24: guide cylinder
- 25: guide needle holding portion
- 26: guide needle
- 27: split piece
- 28: protrusion
- 29: protrusion
- 30: measuring unit
- 31: control unit
- 32: memory unit
- 33: communication unit
- 34: mobile device
- A, B, C, D, E, F: joints

## Claims

1. A biological information measurement device (6), comprising:
an adhesive tape (8) that has on its bottom side an adhesive surface to be affixed to a skin (5) of a user;
a mounting substrate (13) that is disposed on a top side of the adhesive tape (8);
a substrate holding portion (18) that is provided on the top side of the adhesive tape (8) and that is fixed to a top side of the mounting substrate (13), the mounting substrate (13) being held on an outer periphery of the substrate holding portion (18);
a sensor (17) that is electrically connected connected to the mounting substrate (13) and is configured to measure biological information;
a cover (19) that covers a top side of the mounting substrate (13),
spacers (16) disposed between a lower surface of the mounting substrate (13) and an upper surface of the adhesive tape (8), and
a base (12) that is disposed between the adhesive tape (8) and the mounting substrate (13), and that is substantially annular in shape;
wherein a bottom side of the mounting substrate (13) is in a state of not being affixed to the top side of the adhesive tape (8),
the adhesive tape (8) and the cover (19) are more stretchable than the mounting substrate (13),
the spacers (16) are bonded to the lower surface of the mounting substrate (13) on a top side, and are not bonded to the adhesive tape (8), and
the adhesive tape (8) and the cover (19) are bonded together near an outer peripheral edge portion (A), with the base (12) interposed therebetween.

2. The biological information measurement device (6) according to claim 1,
wherein the mounting substrate (13) has no stretchability in a direction substantially parallel to a surface of the skin (5) in a state in which the adhesive tape (8) has been applied to the skin (5).

3. The biological information measurement device (6) according to claim 1,
wherein the mounting substrate (13) has a plurality of substantially fan-shaped parts, and
the spacers (16) are substantially fan-shaped to match a shape of the mounting substrate (13).

4. The biological information measurement device (6) according to claim 1 or 3,
wherein the base (12) has a substantially annular outer peripheral circle portion (9), an inner peripheral circle portion (10) that is disposed concentrically in a radial direction with the outer peripheral circle portion (9), and a plurality of bridges (11) that connect the outer peripheral circle portion (9) and the inner peripheral circle portion (10) at substantially equal angular intervals.

5. The biological information measurement device (6) according to any of claims 1 to 4,
wherein the substrate holding portion (18) has a substantially cylindrical shape, and the lower surface of the cover (19) is bonded to the substantially cylindrical upper end surface of the substrate holding portion (18), near a central portion of the cover (19).

6. The biological information measurement device (6) according to any of claims 1 to 5, further comprising:
an overtape (2) that is bonded to an upper surface of the cover (19); and
a protrusion (29) that is provided to a portion of the overtape (2) corresponding to the substrate holding portion (18), and is inserted into the substrate holding portion (18).

7. The biological information measurement device (6) according to any of claims 1 to 6, further comprising:
electronic components (14) mounted on the top side of the mounting substrate (13); and
a waterproof film (21) that covers the mounting substrate (13) and the electronic components (14).

## Patentansprüche

1. Messvorrichtung (6) für biologische Information, mit:
einem Klebestreifen (8), der auf seiner Unterseite eine Klebeoberfläche aufweist, die an einer Haut (5) eines Benutzers anzubringen ist,
einem Anbringsubstrat (13), das auf einer Oberseite des Klebestreifens (8) angeordnet ist,
einem Substrathalteabschnitt (18), der auf der Oberseite des Klebestreifens (8) vorgesehen ist und der an einer Oberseite des Anbringsubstrats (13) fixiert ist, wobei das Anbringsubstrat (13) an einer äußeren Peripherie des Substrathalteabschnitts (18) gehalten ist,
einem Sensor (17), der elektrisch mit dem Anbringsubstrat (13) verbunden ist und der ausgestaltet ist, biologische Information zu messen,
einer Abdeckung (19), die eine Oberseite des Anbringsubstrats (13) bedeckt,
Abstandshaltern (16), die zwischen einer unteren Oberfläche des Anbringsubstrats (13) und einer oberen Oberfläche des Klebestreifens (8) angeordnet sind, und
einer Basis (12), die zwischen dem Klebestreifen (8) und dem Anbringsubstrat (13) angeordnet ist und die in ihrer Form im Wesentlichen ringförmig ist,
wobei eine Unterseite des Anbringsubstrats (13) in einem Zustand ist, in dem sie nicht an der Oberseite des Klebestreifens (8) angebracht ist,
der Klebestreifen (8) und die Abdeckung (19) eine größere Streckfähigkeit als das Anbringsubstrat (13) haben,
die Abstandshalter (16) mit der unteren Oberfläche des Anbringsubstrats (13) auf einer Oberseite verbunden sind und nicht mit dem Klebestreifen (8) verbunden sind und
der Klebestreifen (8) und die Abdeckung (19) miteinander in der Nähe eines äußeren Peripheriekantenabschnitts (8) verbunden sind, wobei die Basis (12) dazwischen eingeschlossen ist.

2. Messvorrichtung (6) für biologische Information nach Anspruch 1,
wobei das Anbringsubstrat (13) in einem Zustand, bei dem der Klebestreifen an der Haut (5) angebracht ist, keine Streckfähigkeit in einer Richtung im Wesentlichen parallel zu einer Oberfläche der Haut (5) aufweist.

3. Messvorrichtung (6) für biologische Information nach Anspruch 1,
wobei das Anbringsubstrat (13) mehrere im Wesentlichen fächerförmige Teile aufweist und
die Abstandshalter (16) im Wesentlichen fächerförmig sind, um zu einer Form des Anbringsubstrats (13) zu passen.

4. Messvorrichtung (6) für biologische Information nach Anspruch 1 oder 3,
wobei die Basis (12) einem im Wesentlichen ringförmigen äußeren Peripheriekreisabschnitt (9), einen inneren Peripheriekreisabschnitt (13), der konzentrisch in radialer Richtung mit dem äußeren Peripheriekreisabschnitt (9) angeordnet ist, und mehrere Brücken (11) aufweist, die den äußeren Peripheriekreisabschnitt (9) und den inneren Peripheriekreisabschnitt (10) in im Wesentlichen gleichen Winkelintervallen verbinden.

5. Messvorrichtung (6) für biologische Information nach einem der Ansprüche 1 bis 4,
wobei der Substrathalteabschnitt (18) eine im Wesentlichen zylindrische Form aufweist und
die untere Oberfläche der Abdeckung (19) mit der im Wesentlichen zylindrischen oberen Endoberfläche des Substrathalteabschnitts (18) in der Nähe eines Zentralabschnitts der Abdeckung (19) verbunden ist.

6. Messvorrichtung (6) für biologische Information nach einem der Ansprüche 1 bis 5, ferner mit:
einem Overtape (2), das mit einer oberen Oberfläche der Abdeckung (19) verbunden ist, und
einem Vorsprung (29), der an einem Abschnitt des Overtapes (2) entsprechend zum Substrathalteabschnitt (18) vorgesehen ist und der in den Substrathalteabschnitt (18) eingefügt ist.

7. Messvorrichtung (6) für biologische Information nach einem der Ansprüche 1 bis 6, ferner mit:
elektronischen Komponenten (14), die an der Oberseite des Anbringsubstrats (13) angebracht sind, und
einem wasserdichten Film (21), der das Anbringsubstrat (13) und die elektronischen Komponenten (14) bedeckt.

## Revendications

1. Dispositif de mesure d'information biologique (6) comprenant:
un ruban adhésif (8) qui présente sur sa face inférieure une surface adhésive à attacher sur une peau (5) d'un utilisateur,
un substrat de montage (13) qui est disposé sur une face supérieure du ruban adhésif (8),
une portion de maintien de substrat (18) qui est prévue sur la face supérieure du ruban adhésif (8) et qui est fixée à une face supérieure du substrat de montage (13), le substrat de montage (13) étant maintenu sur une périphérie extérieure de la portion de maintien de substrat (18),
un capteur (17) qui est connecté électriquement au substrat de montage (13) et qui est configuré pour mesurer de l'information biologique,
une couverture (19) qui recouvre une face supérieure du substrat de montage (13),
des espaceurs (16) qui sont disposés entre une surface inférieure du substrat de montage (13) et une surface supérieure du ruban adhésif (8), et
une base (12) qui est disposée entre le ruban adhésif (8) et le substrat de montage (13), et qui est de forme pour l'essentiel annulaire,
dans lequel une face inférieure du substrat de montage (13) est dans un état où elle n'est pas fixée à la face supérieure du ruban adhésif (8),
le ruban adhésif (8) et la couverture (19) sont plus extensibles que le support de montage (13),
les espaceurs (16) sont reliés à la surface inférieure du substrat de montage (13) sur une face supérieure, et ne sont pas reliés au ruban adhésif (8), et
le ruban adhésif (8) et la couverture (19) sont reliés l'un à l'autre à proximité d'une portion de bord périphérique extérieure (A), la base (12) étant interposée entre eux.

2. Dispositif de mesure d'information biologique (6) selon la revendication 1,
dans lequel le substrat de montage (13) n'a pas d'extensibilité dans une direction pour l'essentiel parallèle à une surface de la peau (5) dans un état dans lequel le ruban adhésif (8) a été appliqué sur la peau (5).

3. Dispositif de mesure d'information biologique (6) selon la revendication 1,
dans lequel le substrat de montage (13) comprend une pluralité de parties pour l'essentiel en forme d'éventail, et
les espaceurs (16) sont pour l'essentiel en forme d'éventail pour s'ajuster à une forme du substrat de montage (13).

4. Dispositif de mesure d'information biologique (6) selon la revendication 1 ou 3,
dans lequel la base (12) présente une portion de cercle périphérique externe pour l'essentiel annulaire (9), une portion de cercle périphérique interne (10) qui est disposée de manière concentrique dans une direction radiale avec la portion de cercle périphérique externe (9), et une pluralité de ponts (11) qui relient la portion de cercle périphérique externe (9) et la portion de cercle périphérique interne (10) à des intervalles angulaires sensiblement égaux.

5. Dispositif de mesure d'information biologique (6) selon l'une quelconque des revendications 1 à 4,
dans lequel la portion de maintien de substrat (18) présente une forme pour l'essentiel cylindrique, et
la surface inférieure de la couverture (19) est reliée à la surface d'extrémité supérieure pour l'essentiel cylindrique de la portion de maintien de substrat (18), à proximité d'une portion centrale de la couverture (19).

6. Dispositif de mesure d'information biologique (6) selon l'une quelconque des revendications 1 à 5, comprenant en outre:
un sur-ruban (2) qui est relié à une surface supérieure de la couverture (19), et
une saillie (29) qui est prévue sur une portion du sur-ruban (2) correspondant à la portion de maintien de substrat (18), et qui est insérée dans la portion de maintien de substrat (18).

7. Dispositif de mesure d'information biologique (6) selon l'une quelconque des revendications 1 à 6, comprenant en outre:
des composants électroniques (14) qui sont montés sur la face supérieure du substrat de montage (13), et
un film étanche à l'eau (21) qui recouvre le substrat de montage (13) et les composants électroniques (14).
